(19) Europäisches Patentamt
European Patent Office
Office européen des brevets

(11) EP 4 364 759 A1

# (12) EUROPEAN PATENT APPLICATION

(43) Date of publication:
**08.05.2024 Bulletin 2024/19**

(21) Application number: **22306657.2**

(22) Date of filing: **03.11.2022**

(51) International Patent Classification (IPC):
***A61L 15/32*** *(2006.01)* ***C08H 1/02*** *(2006.01)*
***C08H 1/06*** *(2006.01)* ***C08J 3/24*** *(2006.01)*

(52) Cooperative Patent Classification (CPC):
**C08J 3/246; A61L 15/32; A61L 15/325; A61L 15/60; C08H 1/06; C08L 89/06;**
C08J 2300/208; C08J 2305/08; C08J 2389/06

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Université Côte d'Azur**
  **06100 Nice (FR)**
- **Centre national de la recherche scientifique**
  **75016 Paris (FR)**
- **Université Claude Bernard Lyon 1**
  **69100 Villeurbanne (FR)**

(72) Inventors:
- **GUIGO, Nathanaël**
  **06640 La Gaude (FR)**
- **MEHIRI, Mohamed**
  **06000 Nice (FR)**
- **MELILLI, Giuseppe**
  **06000 Nice (FR)**
- **ROUSSELLE, Patricia**
  **69367 Lyon (FR)**
- **SBIRRAZZUOLI, Nicolas**
  **06310 Beaulieu-sur-Mer (FR)**

(74) Representative: **LLR**
**2, rue Jean Lantier**
**75001 Paris (FR)**

## (54) CROSSLINKED POLYMERS AND THEIR USES

(57) The invention relates to a composition comprising a homogenous mixture:
- a polymer containing at least two free amino functional groups, and
- a compound comprising an aldehyde functional group, said compound being of formula I

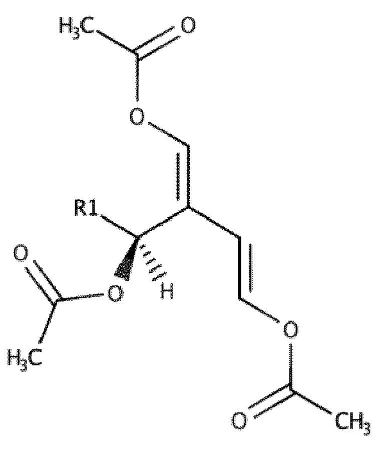

(I);

or of formula II,

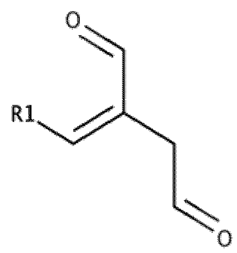

(II),

wherein R1 is C5-C25 alkyl, having one or more double bonds, and/or one or more triple bonds, possibly branched,
wherein the stoichiometric ratio between the aldehyde functional group contained in the compound and the amino functional groups contained in the polymer is from 0.1 to 3.

EP 4 364 759 A1

## Description

The invention relates to crosslinked polymers, and their uses in particular in therapy and treatments of human and animal body.

Tissue engineering involves the use of materials with suitable biochemical and physiological properties to replace, repair, regenerate biological tissues and/or enhance these processes. The particular tissue involved may have certain mechanical and structural requirements for proper functioning. There is a need for materials which are easily tunable for use with a particular target tissue and have suitable biochemical and physiological properties for tissue engineering.

Polymer hydrogels have structural similarities to numerous macromolecular components in the human body, are generally considered biocompatible, and have been investigated extensively as materials useful for drug delivery, tissue repair and tissue engineering, as well as for use as surgical sealants and adhesives. With increasing frequency, polymer hydrogels are designed for in situ gelation from a liquid precursor, thereby allowing minimally invasive administration via syringe or needle.

Existing hydrogel systems, formed chemically or physically, are subject to several limitations. Chemically cross-linked hydrogels often employ toxic cross-linking agents and/or radicals, and the resulting hydrogels are often non-biodegradable. On the other hand, physical hydrogels -- formed through ionic interactions, hydrophobic interactions, hydrogen bonding, or phase transition -- are relatively weak and can be prone to unwanted or uncontrollable degradation through ion exchange, ion diffusion, or monomer dissolution. An alternate approach --solidification by enzymatic cross-linking -- has two principal advantages, compared to other hydrogel systems. First, an enzyme has substrate specificity to allow controllable gel formation. Second, an enzymatic method can be applied to the in vivo utilization of cross-linked hydrogels, under appropriate physiological conditions.

A particular biopolymer for use in medical applications is disclosed in U.S. patent. No. 6,132,759, which relates to a medicament containing a biopolymer matrix comprising gelatin cross-linked with oxidized polysaccharides. The biopolymer of this patent is claimed to be useful for treating skin wounds or dermatological disorders.

However, use of non-natural crosslinker is not required.

Biomass and biomass-derived products have attracted a lot of attention as potential precursors for a wide variety of bio-based materials, but also for the appeal of natural platform molecules that can also offer a great combination of strategies to design innovative bioinspired functional materials. Among the different algal bioresources, some of them - mainly macroalgae - are still under-exploited while they can be highly invasive, which can lead to ecological problems.

Therefore, there is a need to provide new bio-based materials, useful for animal and human application, avoiding chemistry based-compound.

One aim of the invention is to provide a new and easy process to produce bio-based gel, that is biocompatible for animal and human application.

Other aim of the invention is to provide medical and non-medical application of this gel.

Still another aim of the invention is to provide a process for producing such a gel.

Thus, the invention relates to a composition, preferably isolated, comprising a homogenous mixture of:

- a polymer containing at least two free amino functional groups, and
- a compound comprising an aldehyde functional group, said compound being of formula I

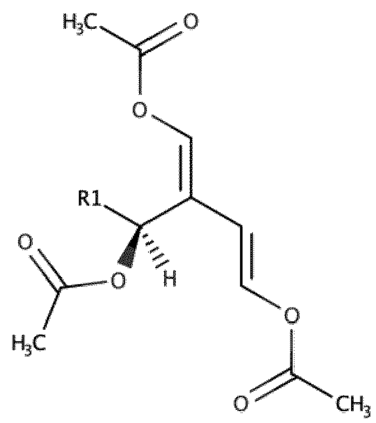

(I);

or of formula II,

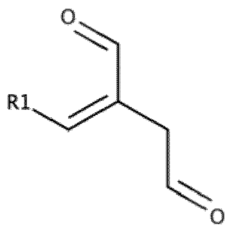

(II),

wherein R1 is C5-C25 alkyl, having one or more double bonds, and/or one or more triple bonds, possibly branched,
wherein the stoichiometric ratio between the aldehyde functional group contained in the compound and the amino functional groups contained in the polymer is from 0.1 to 3.

The invention is based on the unexpected observation made by the inventors that a composition comprising a polymer having free amino functional groups and the compound of formula I or II, can form, further to crosslinking, reaction a new and useful crosslinked polymer that can be used for many applications, and in particular for medical applications.

Due to the reaction between free amino functional groups and aldehyde functional groups, a Paal-Knorr reaction is carried out, creating a covalent bond between the polymer and the compound of formula I or II.

The reaction, because the polymer contains at least two free amino functional groups, will produce a crosslinked network, on the contrary to a reaction involving a polymer having only one free amino functional group, which lead to crosslinked fiber or linear polymer.

In the invention, "free amino functional groups" means a primary, a secondary or a tertiary amine, preferably primary amine, which is available to react with an aldehyde. This free amino functional group is therefore exposed at the periphery of the polymer, for instance as the final functional group of a ramified group of a polymer.

In the invention, "an aldehyde functional group" means an aldehyde group of formula -CHO or -CH=O, called free aldehyde, or a solvate or a ionic form thereof, or any protected aldehyde group wherein oxygen atom is protected by acetalization, or other kind of protection such as : dithiane, hemiacetal, or acetal.

The aldehyde functional group according to the invention, in its protected form, can be obtained directly from plan or animal (i.e. the protection occurs naturally), or protected by a chemical reaction carried out in a laboratory.

Compounds of formula I and II can be considered, within the context of the invention, as crosslinking agents. They are involved in the resulting crosslinked network and are not considered as a catalyst of the reaction.

Both compounds of formula I and II have a residue R1 which is a C5-C25 alkyl, which means that R1 is a C5, C6, C7, C8, C9, C10, C11, C12, C13, C14, C15, C16, C17, C18, C19, C20, C21, C22, C23, C24 or C25 alkyl comprising one or more double bond and/or one or more triple bond.

Examples of R1 residue according to the invention are advantageously C5-C9 alkyls as defined above, and more preferably the following residues:

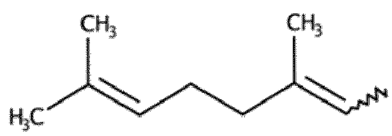

,

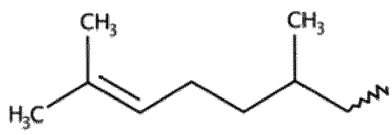

,

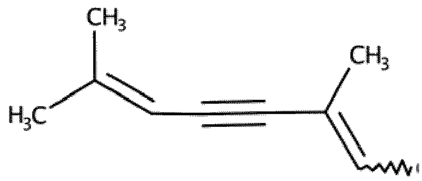

,

and

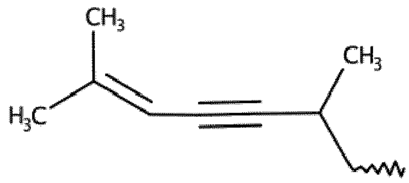

.

Compounds of formula I and II harbor at least 2 aldehyde functional groups, each of these at least 2 aldehyde functional groups being able to react, as defined above, with a free amino functional group. Within the context of the invention, aldehyde means a -CO-H group, i.e. an aldehyde per se, but also an acetaldehyde group of formula $-CO-CH_3$.

Advantageous compounds of formula I are the following ones:

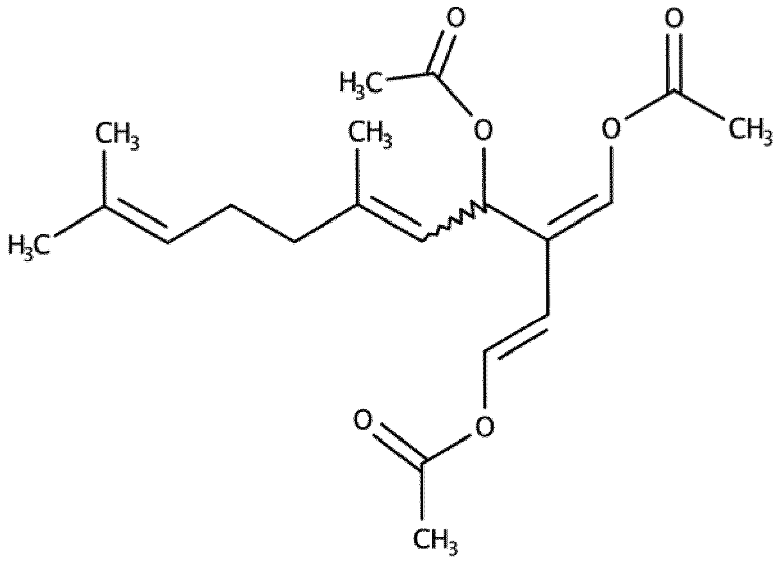

,

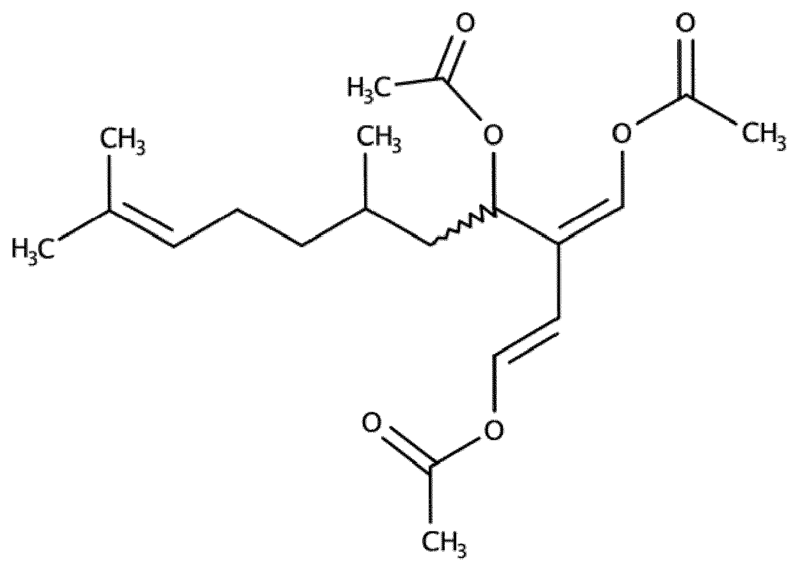

,

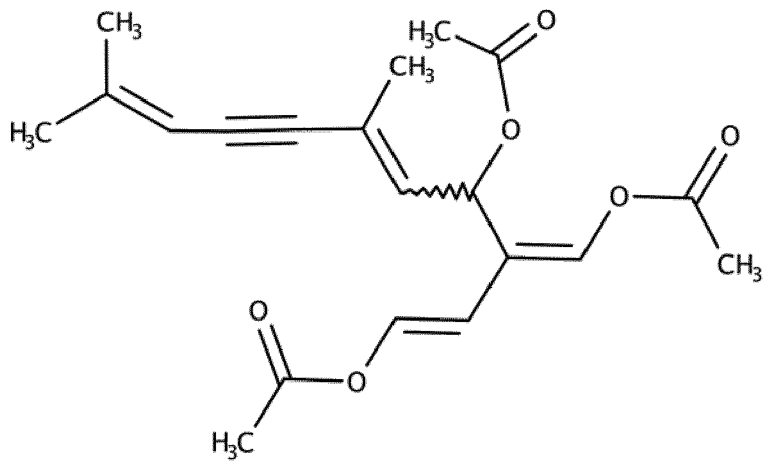

,

and

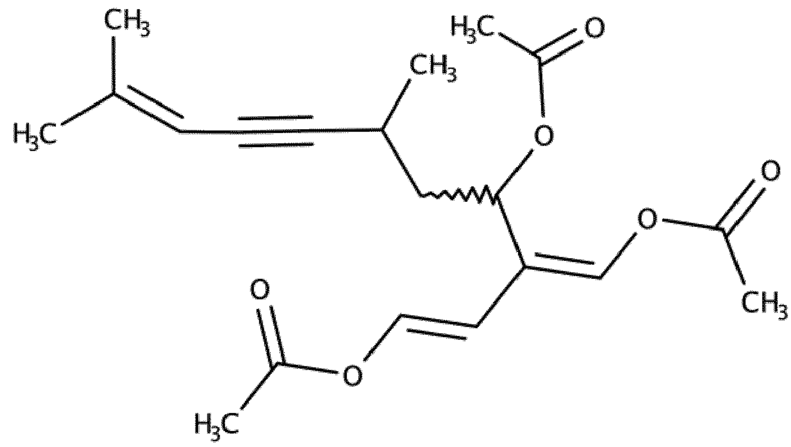

.

Advantageous compounds of formula II are the following ones:

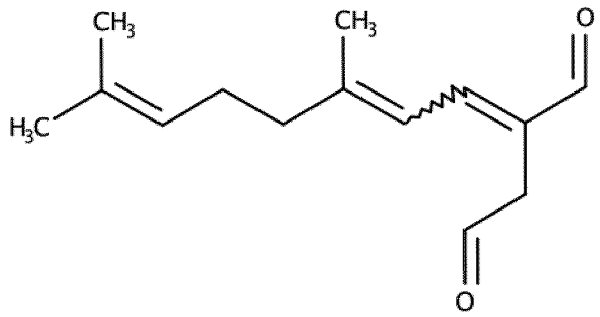

,

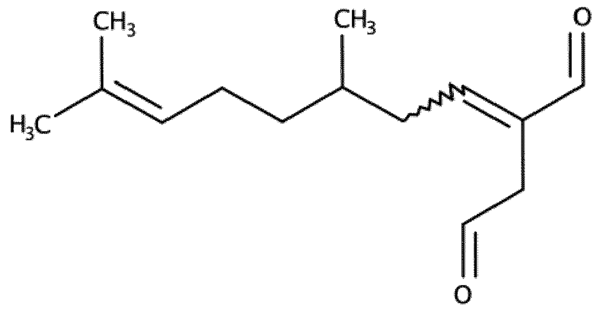

,

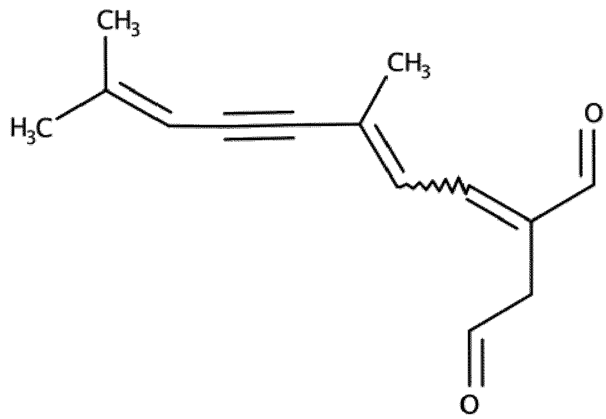

,

and

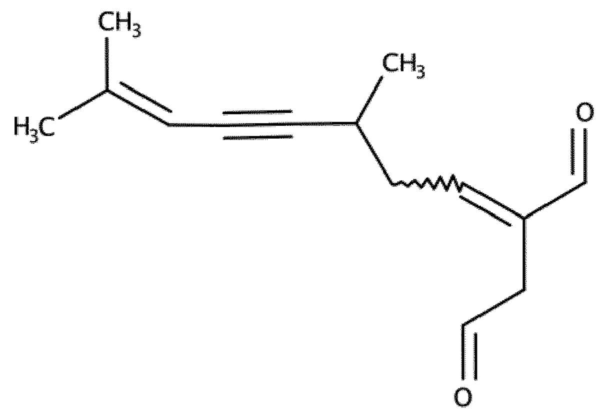

.

The composition according to the invention, the polymer and the compounds I or II are chosen such that the stoichiometric ratio between the aldehyde functional groups contained in the compound and the amino functional groups

contained in the polymer is from 0.1 to 3. This means that the nature and/or the amount of the polymer will determine the amount of compound I or II in the composition.

A stoichiometric ratio shows the relationship between the molecules present in an equation. These proportion convey the necessary quantities of certain chemical functional groups with the intent to trigger the chemical reactions.. When the ratio of the aldehyde functional groups contained in the compound and the amino functional groups contained in the polymer equals to 0.1, this means that the composition comprises 1 mole of aldehyde functional groups contained in the compound and 10 moles of the amino functional groups contained in the polymer. Moreover, when the ratio of the aldehyde functional groups contained in the compound and the amino functional groups contained in the polymer equals to 3, this means that the composition comprises 3 moles of aldehyde functional groups contained in the compound and 1 mole of the amino functional groups contained in the polymer.

In the invention, a stoichiometric ratio varying from 0.1 to 3 means that the ratio can be, for example, equal to 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9 or 3. According to the above examples, the skilled person is able to determine the proportion of the aldehyde functional groups and the amino functional groups.

The invention also relates to a structured network, preferably which is 3-dimentional, comprising or consisting essentially of a polymer containing at least two free amino functional groups operably crosslinked to compound comprising two aldehyde groups of formula II,

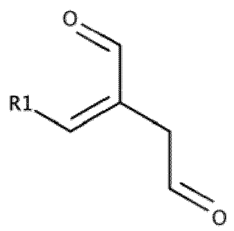

(II),

wherein R1 is as defined above, and
wherein the stoichiometric ratio between the aldehyde functional group contained in the compound and the amino functional groups contained in the polymer is from 0.1 to 3.

In another aspect the invention is based on the observation made by the inventors that the mixture of polymer containing at least two free amino functional groups with a compound of formula II produce a crosslinking reaction. A network is therefore obtained with specific features as described below.

The network obtained from the crosslinking reaction mentioned above is also called in the invention a scaffold.

The structured network according to the invention is either in a dry form or an hydrated form.

This network, or scaffold, or matrix, is a tridimensional network wherein chains of polymers as defined above are connected to each other via compounds of formula II. This connection between the polymer and the compound of formula II is defined as an operably linkage and it corresponds to a covalent linkage between the molecules.

In other words, in the network according to the invention, polymer chains are covalently linked to molecules of compounds of formula II, in order to form a tridimensional structure.

As discussed earlier, the connection between the polymer and the compound II is carried out by a reaction between an aldehyde of compound and an amino group of the polymer. A majority of the bond created during the crosslinking reaction are intermolecular bonds, i.e. a compound of formula II connect to each other two different molecules of polymer. However, some intermolecular bonds can also be present in the network described herein, i.e. a compound of formula II connected to different amino functional group of the same unit of polymer.

Since the structure resulting of this crosslinking reaction is a network, the main bonds, i.e. the main covalent bonds, are intermolecular bonds.

Since the polymer and the compound of formula II are covalently linked, the structure is rigid, which means that only high molecular force (or high energy) can break the bonds between the polymer and the compound.

The network or scaffold described herein is therefore a structure containing interconnected open pores or interconnected voids, that could contain, or capture or absorb, a liquid, preferably an aqueous liquid. Advantageously, the pore size is in the range of 0.1 to 1000 μm, preferably of 0,1 to500 μm. The pore number and size will depend upon the ratio between the aldehyde functional group contained in the compound and the amino functional groups contained in the polymer. If the ratio is close to 3, the pore number will be reduced compared to a network wherein the ratio is close to 0.1. Inversely, If the ratio is close to 3, the size of the pore will be larger compared to the network having a ratio close to 0.1.

As such, the network according to the invention is dried, and is only constituted of the polymer and the compound of formula 2. However, in presence of an aqueous liquid, said liquid will be able to occupy the voids or pores within the polymer such that the network becomes or remains wet. In other words, the voids or pores within the polymer are able to absorb said liquid to hydrate the network.

The network according to the invention is able to absorb more than 30 times its initial dry weight in aqueous liquid, in particular when the aqueous liquid is distilled water.

More advantageously, the invention relates to the structured network above mentioned, wherein the polymer containing at least two free amino functional groups is an animal or plant protein, a polysaccharide containing an amino group or an artificial amino polymer.

Advantageously, the polymer is a natural polymer originated from plant or animal, the polymer being chosen from amino polysaccharides such as chitosan, or amino modified polysaccharides, proteins containing amino acids such as lysine, arginine, histidine, ornithine, or peptides constituted by a repletion of one or more of these amino acids, such as poly-lysine peptides, poly-arginine, poly-ornithine, or poly histidine.

The polymer can also be an artificial, i.e. non naturally occurring polymer, Such as polyethylene glycol diamine and poly (vinyl) amine.

In the invention, if the polymer is originating from green algae, such as *Caulerpa taxifolia* algae, thus the compound of formula II involved in the crosslinking of the polymer - in order to produce the network defined above - is not Caulerpenyne (CYN) compound of formula IV

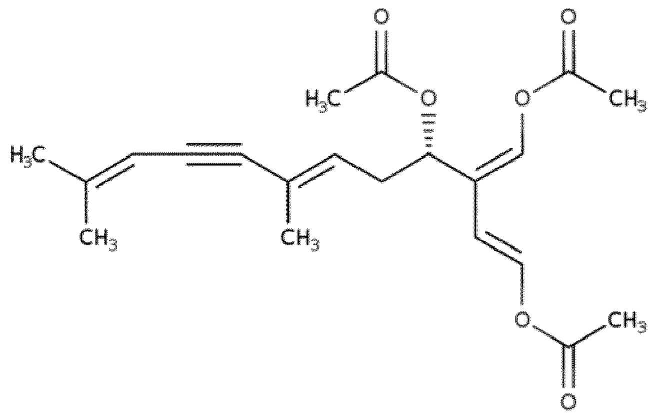

(IV)

or oxytoxine compound of formula III

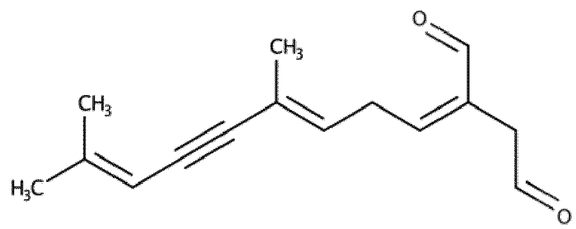

(III).

More advantageously, the invention relates to the structured network as defined above, wherein the animal or plant protein is chosen from gelatin, keratin, casein and lactoserum proteins, elastin, collagen, soy protein, serum bovine albumin, fibrin and fibrinogen, and resilin.

Collagen is the main structural protein in the extracellular matrix found in the body's various connective tissues. As the main component of connective tissue, it is the most abundant protein in mammals. Collagen consists of amino acids bond together to form a triple helix of elongated fibril known as a collagen fibers.

Gelatin is a denatured product of collagen under acid, alkali, enzyme or high-temperature conditions. It is partially hydrolyzed to hold on to a lot of water causing gelling.

Keratin is one of a family of structural fibrous proteins also known as scleroproteins. Alpha-keratin (a-keratin) is a type of keratin found in vertebrates. Keratin monomers assemble into bundles to form intermediate filaments, which are tough and form strong unmineralized epidermal appendages found in reptiles, birds, amphibians, and mammals.

Casein is a family of related phosphoproteins ($\alpha$S1, aS2, $\beta$, $\kappa$) that are commonly found in mammalian milk,

comprising about 80% of the proteins in cow's milk.

Lactoserum, or whey is the liquid remaining after milk has been curdled and strained. Whey proteins consist of α-lactalbumin, β-lactoglobulin, serum albumin, immunoglobulins, and proteose peptones.

Bovine serum albumin corresponds to bovine serum "Fraction V", i.e. the fifth fraction of the original Edwin Cohn purification methodology that made use of differential solubility characteristics of plasma proteins.

Elastin is a protein that is a key component of the extracellular matrix in gnathostomes (jawed vertebrates). Present in connective tissue, elastin allows many tissues to resume their shape after stretching or contracting.

Soy protein is a protein that is isolated from soybean. It is made from soybean meal that has been dehulled and defatted.

Fibrinogen (factor I) is a glycoprotein complex, produced in the liver, that circulates in the blood of all vertebrates. During tissue and vascular injury, it is converted enzymatically by thrombin to fibrin and then to a fibrin-based blood clot. The fibrinogen molecule circulates as a soluble plasma glycoprotein with a typical molecular weight of about 340- about 420 kDa

Fibrin (also called Factor Ia) is a fibrous, non-globular protein involved in the clotting of blood. It is formed by the action of the protease thrombin on fibrinogen, which causes it to polymerize.

Resilin is an elastomeric protein found in many insects and other arthropods. It provides soft rubber-elasticity to mechanically active organs and tissue

More advantageously, the polysaccharide containing an amino group is chosen from chitosan and, amino-hyaluronic acid, naturally aminated carbohydrates, but also mino-functionalized carbohydrates or sugar, such as amino-functionalized cellulose, amino-functionalized agarose, amino- functionalized alginate, amino- functionalized heparin and amino- functionalized chondroitin sulphate.

Chitosan is a linear polysaccharide composed of randomly distributed β-(1→4)-linked D-glucosamine (deacetylated unit) and N-acetyl-D-glucosamine (acetylated unit).

Hyaluronic acid, also called hyaluronan, is an anionic, nonsulfated glycosaminoglycan distributed widely throughout connective, epithelial, and neural tissues. Hyaluronic acid is
constituted by a repetition β (1→4) of a dimer of D-Glucuronic acid and N-acetyl-D-glucosamine (1→3)).

More advantageously, an artificial amino polymer encompassed by the invention is chosen from polyethylene glycol diamine and poly (vinyl amine).

More advantageously, the invention relates to the structured network as defined above, the compound of formula II is oxytoxin-2 of formula III

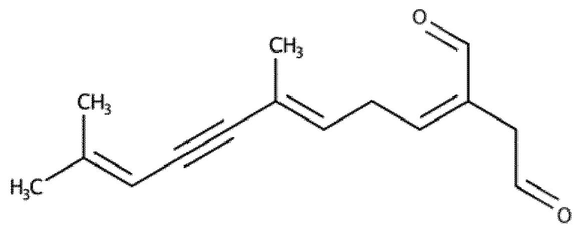

(III).

In this advantageous embodiment, the network according to the invention will therefore be constituted by a polymer having free functional amino group, the animo group of said bolymer being linked to oxytoxin-2 via the aldehyde groups of said compound. If the free amino functional group of the polymer is a primary amine group, then an imine function (-N=CH-) will be the result of the crosslinking, i.e.
polymer-$NH_2$ + O=CH-compound 2 → Polymer-N=CH-compound 2 + $H_2O$, as illustrated hereafter:

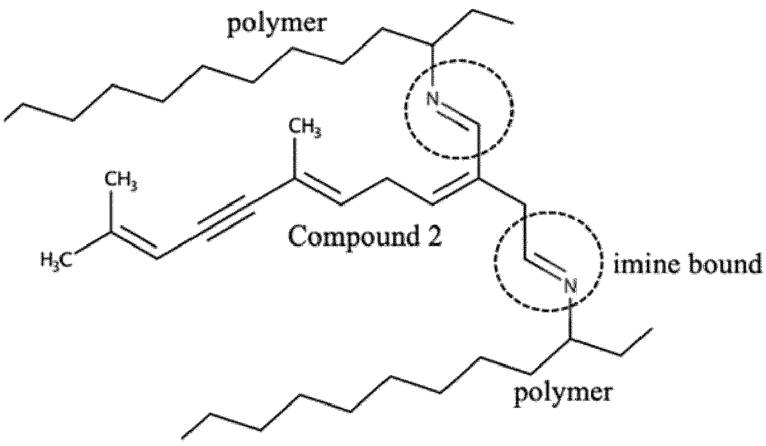


Another mechanism is a crosslinking involving Paal-Knor reaction as illustrated hereafter

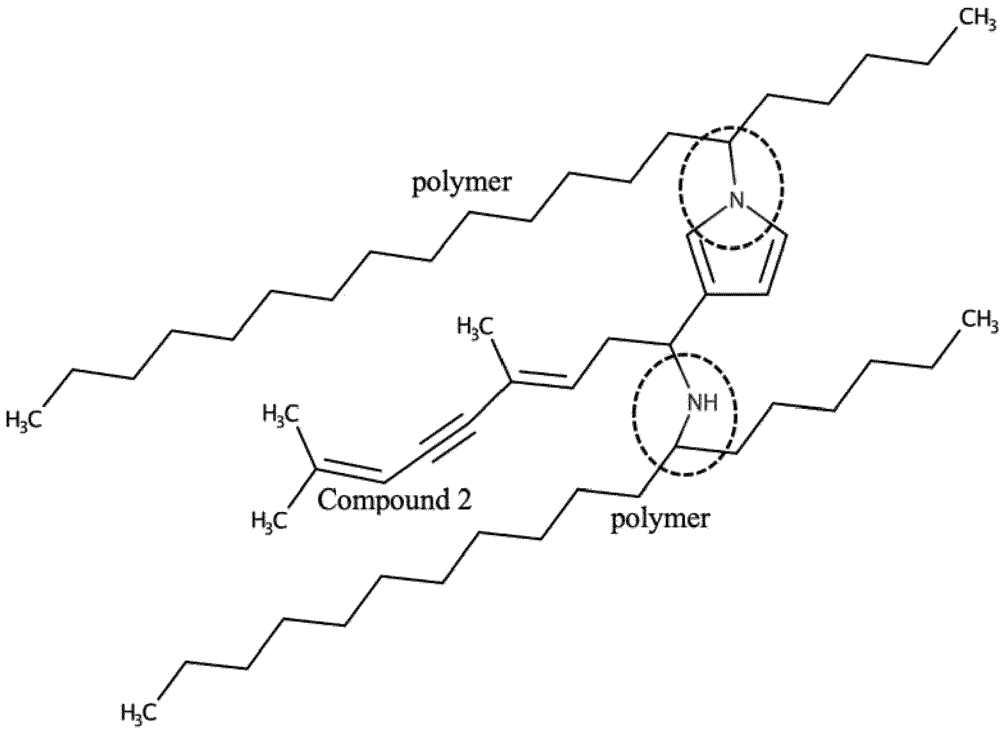


In the invention, reaction between free amino functional groups and aldehyde groups can be either intermolecular reactions, or intermolecular reactions (crosslinking compound reacts with many amino functional group within the same polymer).

Thus, the resulting network according to the invention may contain both types of intermolecular an intramolecular reaction.

The network according to the invention can also corresponds to a superposition of polymers that have reacted in an intramolecular manner with the crosslinking agent.

More advantageously, the invention relates to the structured network as defined above, wherein the polymer containing at least two free amino functional groups is chosen from gelatin and chitosan.

Advantageously, the network according to the invention is constituted by oxytoxin-2 and gelatin.

Advantageously, the network according to the invention is constituted by oxytoxin-2 and chitosan.

The invention also relates to the use of a composition as defined above, or a structured network as defined above, for lubricating a hydrophobic surface of a device, in particular a medical device.

In the invention, the network as defined above can be used in order to cover an hydrophobic surface of a medical device, in order to lubricate this surface.

Medical devices can be covered with a multitude of compositions or compounds depending on the intended uses. It may in particular be necessary to cover a medical device with a composition comprising a pharmaceutical agent or an agent allowing hydration, in particular in the context of implantation in an organism. Likewise, the use of a medical device can be improved by covering it with a lubricating composition, i.e. the network according to the invention, preferably in an hydrated form.

Therefore, in the invention, the "surface" or the "support" corresponds to a solid medical device, in particular that which is likely to cause friction while penetrating the human or animal body. For example, without being limiting, the support may be: the inside of a syringe, the plunger of a syringe, the rubber of the plunger of a syringe, a hollow needle, whether or not used with a syringe (whether it is acts on the outer surface of the needle or the channel thereof), a stent, a catheter (in particular for intracerebral, intracardiac, and vascular use as well as vascular guide wires), a lens (in particular a lens intended to be inserted into the eye), a probe, a drill, and more generally any medical or surgical instrument intended to allow the placement of an implant, a prosthesis suitable for being inserted into the human or animal body temporarily or permanently, a heart valve, a pacemaker, in particular an artificial pacemaker, an orthopedic implant, etc.

In the invention, the term "medical device" takes the legal meaning defined by French law, namely any instrument, device, equipment, material, product, or manufactured product, with the exception of products of human or animal origin, or any other article used alone or in combination, intended by the manufacturer for medical use in humans and the desired principal action of which is not obtained by pharmacological or immunological means or by metabolism, but the function of which may be assisted by such means.

The invention also relates to a structured network according as defined above, for its use for treating a human or an animal wound.

As is well-known in the prior art, usual methods for the cure of wounds whether in humans or animals, involve certain well-defined steps. These are curettage (removal of dead/infected tissues), disinfection (with disinfectants containing either iodine or hydrogen peroxide) and antibiotic therapy (either local or systemic, in form of powder, cream or spray). Lastly, to avoid hardening of the skin and crust formation, gauzes soaked in fatty humectants based on Vaseline, silicone oils or glycerol are applied. Dressing of the wound with sterile gauze is usually carried out not only to prevent exposure of the wound to infectious agents e.g. bacteria present in the environment, but also to soak exudations and secretions from the wound. These bandages need to be removed at regular intervals. In poor hygienic or environmental conditions e.g. those frequently present in developing countries, the wounds cannot be managed adequately and remain exposed to dust and environmental infestations which can contribute to important infections of the wound. Despite efficacious anti-microbial treatment and improved supportive measures, wound treatment still poses immense challenges. Treatment and cure for invasive infections, blunt injuries, burns caused by electrical or chemical accidents, radiation burns and the like, is not very effective and leaves much to be desired.

According to the present invention a network as defined above is provided for external or topical application, for the treatment and cure of all types of wounds and lesions in humans and animals. The network exhibits remarkable efficacy in treating and curing wounds, possibly which are regarded as 'incurable' in state of the art.

The network as defined above improves blood flow, tissue growth and has immuno-modulatory properties. The network is also useful for treating infections and chronic non healing wounds like diabetic foot ulcer, dry and wet gangrene, venous ulcer, varicose veins, war wounds, burn wounds, post operative situation and the like.

Another embodiment of the invention is that the network acts by applying it topically e.g skin applications and or externally (gynaecological applications, mucous membranes etc).

The invention also relates to a medical device comprising a surface, the medical device comprising a structured network as defined above coated to the surface.

Advantageously, the invention relates to the medical device as defined above, said medical device being a wound dressing; a patch; a film; a wound filler; an hydrogel dressing; a skin substitute; an hydrocoilloid dressing or a foam. The invention relates to in vitro devices such as a carrier for cells, molecules, genes, bioactive compounds or nanoparticles; and an in vitro scaffold for organoid culture.

Medical devices according to the invention can be used for an implantation in human or animal body, but can also be used in vitro as a coating of culture dishes or a scaffold for organ equivalent, organoid and spheroid tridimentional cultures.

The invention also relates to a method for producing a structured network, preferably a structured 3-dimensional network, the method comprising:

- mixing
  - a first organic solvent, in particular water, comprising a polymer containing at least two free amino functional groups; and
  - a second organic solvent comprising a compound containing comprising 2 aldehyde groups of formula I,

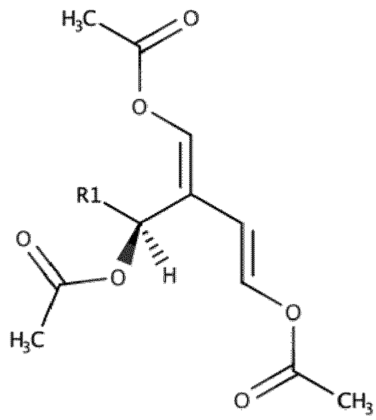

(I),

to obtain a mixture wherein the stoichiometric ratio between the aldehyde functional group contained in the compound and the amino functional groups contained in the polymer is from 0.1 to 3, wherein said first organic solvent is compatible, i.e. miscible, with said second solvent,

- contacting the mixture with a basic composition or solution, to obtain a crosslinked mixture,
- possibly washing the crosslinked mixture, and
- recovering the crosslinked mixture which is in a form of a structured network, possibly 3-dimensional.

Advantageously, the invention relates to a method for producing a structured network, preferably a structured 3-dimensional network, the method comprising:

- mixing
  - a first organic solvent, in particular water, comprising a polymer containing at least two free amino functional groups; and
  - a second organic solvent comprising a compound containing comprising caulerpenyne of formula III,

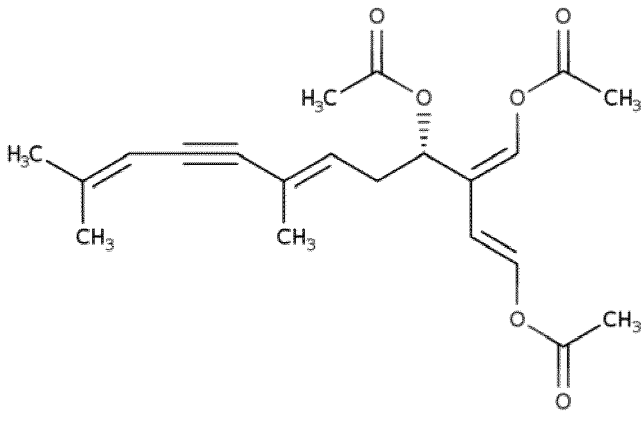

(III),

to obtain a mixture wherein the stoichiometric ratio between the aldehyde functional group contained in the caulerpenyne and the amino functional groups contained in the polymer is from 0.1 to 3, wherein said first organic solvent is compatible, i.e. miscible, with said second solvent,

- contacting the mixture with a basic composition or solution, to obtain a crosslinked mixture,
- possibly washing the crosslinked mixture, and
- recovering the crosslinked mixture which is in a form of a structured network.

In the above process, that allows to obtain the network as defined above, two steps are carried out:

STEP 1: Both polymer and compound as defined above are solubilized in an appropriated solvent, each in respectively in a first and a second solvent, compatible to each other. Then, since the compound harbor protected aldehyde

groups by acetylation. Thus, the use of a basic composition or solution, preferably NaOH, will deprotect the aldehyde group of the compound. If the compound is caulerpenyne, the resulting compound after the basic composition treatment is the production of oxytoxine-2.

STEP 2: this step corresponds to the crosslinking *per se*. Free aldehydes of the compound with therefore react with the free amine contained in the polymer.

Depending upon the stoichiometric ratio, it is possible that some free compounds remain non crosslinked further to the reaction. It could be therefore advantageous to wash the network obtained. This is particularly important for an animal application of the resulting network, when the compound is caulerpenyne, since a toxicity of free caulerpenyne (CYN) has been reported (Parent-Massin (1996), Journal of Toxicology and Environmental Health, 47:1, 47-59).

Advantageously, the invention relates to the method as defined above, wherein the mixture is poured in a recipient and cooled down to a temperature from 2°C to 15°C, preferably 4°C and wherein the polymer containing at least two free amino functional groups is gelatin.

Cool down is carried out during about 3h for jellification of gelatin. The basic composition is then added onto the gel of gelatin.

In view of the crosslinking reaction, it could be advantageous that the solution of polymer and compound be poured into a mold in order to give a predetermined form to the resulting network. Thus, prior to the addition of the basic solution, the mixture is poured on a mold chosen by the skilled person.

More advantageously, the invention relates to the method as defined above, wherein the mixture is contacted with the basic composition at 4°C to 15°C for 2 to 48h, preferably 24h.

The invention relates to a structured network, preferably a 3-dimensional structured network, obtained or liable to be obtained by the method as defined above.

The network is new and is characterized as defined above.

The invention also relates to a kit comprising:

- a polymer containing at least two free amino functional groups as defined above; and
- a compound of formula I

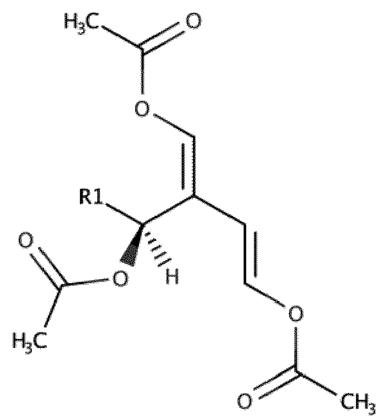

,

Wherein R1 is a C5-C25 alkyl, having one or more double bonds, and/or one or more triple bonds, possibly branched;

- and possibly an aqueous basic solution.

The invention also relates to a compound:

- of formula I as defined above, or
- of formula II as defined above,

for crosslinking, preferably *in vitro* or *ex vivo*, a polymer containing at least two free amino functional groups, in order to obtain a structured network, possibly 3-dimensional, as defined above.

The invention also relates to the use of a compound:

- of formula I as defined above, or
- of formula II as defined above,

for crosslinking, preferably *in vitro* or *ex vivo*, a polymer containing at least two free amino functional groups, in order to obtain a structured network, possibly 3-dimensional.

The cross linker can be used after the 3D printing of gelatin or chitosan.

Already cross-linked gelatin droplets, such as organoids, can be introduced into the ink to be bioprinted.

**Brief description of the drawings**

The invention will be better understood in view of the following drawings and examples

[Fig. 1] **Figure No 1** represent photos of crosslinked gelatin hydrogels obtained with a concentration of 5 wt% (A), 10 wt% (B) or 15 wt% (C) Caulerpenyne (CYN).

[Fig. 2] **Figure No 2** represents microscopic photos of (A) un-crosslinked gelatin, and (B and C) gelatin crosslinked with 5 or 10% Caulerpenyne, respectively. Neat gelatin was processed in the same way without adding CYN. The hydrogel so obtained was referred to uncrosslinked gelatin.

[Fig. 3] **Figure No 3** represents a graph showing the hydrolysis rate (in %) over the time in months, of networks crosslinked with 5 wt% (square), 10 wt% (circle) and 15 wt% (triangle) caulerpenyne (CYN).

[Fig. 4] **Figure No 4** represents a graph showing the sweeling degree (%) of networks crosslinked with 5% (A), 10% (B) and 15% (C) caulerpenyne (CYN).

[Fig. 5] **Figure No 5** is a graph showing fibroblast growth (OD at 590 nm) over the time (in hours) when seeded on plates coated with gelatin (A) or reticulated gelatin with 5 wt% (B - curve with square), 10 wt% (C- curve with triangles) or 15 wt% caulerpenyne.

[Fig. 6] **Figure No 6** represents photographies of human dermal fibroblast seeded on dished under the following conditions:

A: photo of cells after 1h on a dish covered with gelatin reticulated with 5 wt% caulerpenyne,
B: photo of cells after 48h on a dish covered with gelatin reticulated with 5 wt% caulerpenyne,
C: photo of cells after 1h on a dish covered with gelatin reticulated with 10 wt% caulerpenyne,
D: photo of cells after 48h on a dish covered with gelatin reticulated with 10 wt% caulerpenyne,
E: photo of cells after 1h on a dish covered with gelatin reticulated with 15 wt% caulerpenyne,
F: photo of cells after 48h on a dish covered with gelatin reticulated with 15 wt% caulerpenyne,
G: photo of cells after 1h on a dish covered with gelatin, and
H: photo of cells after 48h on a dish covered with gelatin.

[Fig. 7] **Figure No 7** is a graph showing fibroblast adhesion to gels (% of total cells seeded), the gels being A: gelatin, B: gelatin reticulated with 5 wt% caulerpenyne, C: gelatin reticulated with 10 wt% caulerpenyne, D: gelatin reticulated with 15 wt% caulerpenyne.

[Fig. 8] **Figure No 8** is a graph showing human epidermal keratinocytes growth (OD at 590 nm) over the time (in hours) when seeded on plates coated with gelatin (A) or reticulated gelatin with 5 wt% (B), 10 wt% (C) or 15 wt% (D) caulerpenyne.

[Fig. 9] **Figure No 9** represents photographies of human epidermal keratinocytes seeded on dished under the following conditions:

A: photo of cells after 2h on a dish covered with gelatin reticulated with 5 wt% caulerpenyne,
B: photo of cells after 24h on a dish covered with gelatin reticulated with 5 wt% caulerpenyne,
C: photo of cells after 2h on a dish covered with gelatin reticulated with 10 wt% caulerpenyne,
D: photo of cells after 24h on a dish covered with gelatin reticulated with 10 wt% caulerpenyne,
E: photo of cells after 2h on a dish covered with gelatin reticulated with 15 wt% caulerpenyne,
F: photo of cells after 24h on a dish covered with gelatin reticulated with 15 wt% caulerpenyne,
G: photo of cells after 2h on a dish covered with gelatin, and
H: photo of cells after 24h on a dish covered with gelatin.

[Fig. 10] **Figure No 10** is a graph showing human epidermal keratinocytes adhesion to gels (% of total cells seeded), the gels being A: gelatin, B: gelatin reticulated with 5 wt% caulerpenyne, C: gelatin reticulated with 10 wt% caulerpenyne, D: gelatin reticulated with 15 wt% caulerpenyne

## EXAMPLES

### Example 1 - preparation of a gel according to the invention

In this example, it is disclosed a 3D structured network obtained from the crosslinking of gelatin and caulerpenyne.

**STEP1:** Gelatin (20 %w/v) is solubilized in water at a temperature *T*= 60 °C for 3 h.

Then, a mixture Caulerpenyne in ethanol (CYN/EtOH), was added to the solution to achieve the desired CYN concentration (with respect to gelatin). Subsequently, the formulation is poured in a silicon mould and cooled down to *T* = 4 °C for 3h.

**STEP2:** The physical hydrogel containing CYN is then covalently crosslinked in a NaOH solution (0.1 M) for 24 h at *T* = 4 °C. To re-equilibrate the pH and remove any trace of non-reacted CYN the hydrogel was purified in water for 24 h at *T* = 4 °C.

### Example 2 - Properties of the network according to the invention

Examples of gelatin hydrogels obtained with 5, 10, and 15%wt Caulerpenyne. Respectively, 0.053M, 0.107M, and 0.160M of Caulerpenyne solution is added in 2 mL of gelatin aqueous solution (Caulerpenyne. 20%w/v) Caulerpenyne. The abbreviated names for these samples are gel/CYN5, gel/CYN10, and gel/CYN15, respectively.

Macroscopic view are shown in Fig. 1.

Microscopic views of the networks are shown in Fig. 2. Larger porous have been observed for the gel/CYN crosslinked hydrogels compared (B, C) with the gelatin physical (uncrosslinked) (A) network.

The inventors also evaluated the stability of the networks obtained above.

The hydrolysis property of the crosslinked gel/CYN hydrogels was evaluated by immersing them in deionized water for a period of time. Degradation rates of the networks were then assessed. The swelling hydrogels were kept in water and placed in an incubator at 37 °C. At the specified time points of 1,2 months, the remaining hydrogels were obtained, lyophilized, and weighed. The extent of biomaterial hydrolysis was determined by calculating the percentage of remaining weight compared with the original weight.

Results are shown in Fig. 3.

After two months of test, a drastic decrease of the weight for the gel/CYN 5% was observed. The weight loss observed after ageing at 37 °C is inversely proportional to the degree of crosslink.

The Inventors then evaluated the Swelling degree of the networks as described above.

Swelling degree (SD) is a simple method to characterize the water adsorption and stability of the hydrogels. A highly corss-linked polymer shows less degree of swelling. Specimens with cylindrical shape (20 mm in diameter × 10 mm thickness) were prepared and hydrated at 37 °C for 24 h. Subsequently, the samples were taken out from water medium, wiped with tissue paper to remove the excess of water, and weighted. The SD (%) was calculated by the following equation:

[Math 1]

$$SD\,(\%) = \frac{W_{swollen} - W_{dry}}{W_{dry}} * 100$$

*wherein* $W_{swollen}$ was the weight of the swollen sample after 24 h at 37 °C, and $W_{dry}$ the initial weight of the dry sample.

Results are shown in Fig. 4, and in Table 1.

[Table 1]

| Sample | Swelling degree (%) | Standard Deviation |
|---|---|---|
| Gel/CYN5% | **3209** | 1311 |
| Gel/CYN 10% | **1248** | 653 |
| Gel/CYN 15% | **593** | 224 |

Due to its poor hydrolytic resistance, within few minutes after immersion in water the uncrosslinked gelatin hydrogel was completely dissolved.

An increase of the caulerpenyne content leads to decrease the swelling degree. This behaviour demonstrates that a further addition of crosslinking agent (up to 15 %w) increases the covalent linkages between gelatin chains thus decreasing the possibility for a solvent (here water) to swell on polymer chains.

**Example 3 - proliferation and adhesion of cells on the network according to the invention**

In order to evaluate the properties of the network according to the invention on living cells, the inventors evaluated in vitro cell adhesion and growth as a model of dermis regeneration further to injury.

Normal human primary fibroblasts were isolated from human skin as previously described and maintained in culture in DMEM medium containing 10% FCS (fetal calf serum). Cells were detached from the culture plates and $10^5$ fibroblasts were seeded with 100 µl of DMEM 10% FCS (5 wells / condition) in a plate in which the bottom of the wells have been coated with

- gelatin,
- a reticulated network constituted by gelatin and caulerpenyne 5 % wt/v,
- a reticulated network constituted by gelatin and caulerpenyne 10% wt/v, or
- a reticulated network constituted by gelatin and caulerpenyne 15% wt/v.

As control, wells coated with uncrosslinked gelatin were used.

The inventors first evaluated cell survival and growth over the time, further to the cell seeding onto the above-mentioned networks.

After 2h at 37°C in an humidified incubator at 5% $CO_2$, the cell culture medium was removed and replaced with DMEM 10% FCS containing 12,5% alamar Blue cell viability reagent. To monitor cell growth, absorbance of the well supernatants was recorded at 590 nm at different time points during 48h. Fibroblasts were photographed using an Axiovert 40 Zeiss microscope coupled to a Coolsnap Fx Camera (Roper Scientific, Evry, France). Unpaired t tests were used to analyze the data, with a p-value of less than 0.05 considered statistically significant. Statistical analyses were performed with GraphPad Prism software, version 8.4.2 (GraphPad Software). All results are expressed as the mean ± SEM. ****p<0.0001 vs. control, Student's t-test.

Results for cell proliferation, evaluated by Alamar Blue protocol, are shown in Fig. 5.

These results show that cell growth over 48h is not affected when using the network according to the invention and that the gels allow regular growth to reach a living cell layer colonizing the gels. (Fig. 6) This is an advantage as dermal fibroblasts need to proliferate to be able to quickly colonize and fill the entire gel and then produce extracellular matrix to rebuild the dermis.

Then the inventors evaluated cell adhesion of fibroblasts onto the networks according to the invention. Human primary fibroblasts were detached from the culture plates and $4 \times 10^4$ cells were seeded in each well in 100 µl of DMEM (5 wells / condition). After 1h at 37°C in an humidified incubator at 5% the non-adhered cells were collected and counted. The number of adhered cells was deduced by subtracting the non-adhered cells from the total number of seeded cells. One-way ANOVA were used to analyze the data, with a p-value of less than 0.05 considered statistically significant. Statistical analyses were performed with GraphPad Prism software.

Results are shown on Fig. 7.

As expected, after 1 hour, percentage of adherent fibroblasts is the highest on plates coated with a network containing 15% w/v caulerpenyne. Moreover, whatever the concentration of caulerpenyne used, fibroblast adhesion is significantly increased compared to the non-reticulated gelatin (Gelatin)

These results show that the three cross-linked gels allow very rapid and effective adhesion of dermal fibroblasts, which is an advantage for the repair of skin wounds. They also show that the rigidity of the gel impacts this cell adhesion property because the more the gel is cross-linked, the faster the cells attach. This characteristic is an asset that can be exploited depending on the type of wound addressed.

In a second time, the inventors evaluated the properties of the network according to the invention on living cells, the inventors evaluated in vitro cell adhesion as a model of epidermis.

Normal human primary keratinocytes were isolated from human skin and grown in culture in supplemented keratinocyte growth medium containing 0.15 mM CaCl2 (KBM-2 BulletKit, Lonza Biosciences, Basel, Switzerland). Cells were detached from the culture plates and 4 x $10^5$ were seeded with 100 µl of KBM-2 medium in a plate in which the bottom of the wells have been coated with

- gelatin,
- a reticulated network constituted by gelatin and caulerpenyne 5% wt,
- a reticulated network constituted by gelatin and caulerpenyne 10% wt, and
- a reticulated network constituted by gelatin and caulerpenyne 15% wt.

The inventors first evaluated cell survival and growth over the time, further to the cell seeding onto the abovementioned networks.

After 2H at 37°C in an humidified incubator at 5% the cell culture medium was removed and replaced with KBM-2 containing 12,5% alamarBlue cell viability reagent. To monitor cell growth, absorbance of the well supernatants was recorded at 590 nm at different time points during 28h (until cells reached confluency). Keratinocytes were photographed using an Axiovert 40 Zeiss microscope. All results are expressed as the mean ± SEM.

Results for cell viability, evaluated by Alamar Blue protocol, are shown in Fig. 8, and representative photos are shown in Fig. 9.

These results show that keratinocyte growth is compatible with the three cross-linked gels tested. The growth take place gradually and correctly on the surface of the three cross-linked gels to generate confluent cellular sheets resembling the epidermis. Uncrosslinked gelatin, known to be a good substrate for keratinocytes, induces cell hyperproliferation, generating at 24h, clusters of dead cells on the surface of the cell layer. It is noted that the more the gel is cross-linked, the more cell proliferation is controlled leading to a cohesive cell sheet. Being able to control the stiffness of the gel by modulating the cross link and thus modulating keratinocyte growth is a powerful feature. (COMMENTAIRE SUR LES KERATINOCYTE -> PATRICIA). As epidermal regeneration is a resurfacing of the wound, keratinocyte to not enter within the gel but rather migrate and slowly disperse over it. The cross-linked gels allow progressive development of the keratinocyte sheet without inducing the hyperproliferation seen with the uncrosslinked gelatin.

Then the inventors evaluated cell adhesion on keratinocytes onto the networks according to the invention.

Human primary keratinocytes were detached from the culture plates and $4 \times 10^4$ cells were seeded in each well in 100 μl KBM-2 (5 wells / condition). After 1H at 37°C in an humidified incubator at 5% the non-adhered cells were collected and counted. The number of adhered cells was deduced by subtracting the non-adhered cells from the total number of seeded cells. One-way ANOVA were used to analyze the data, with a p-value of less than 0.05 considered statistically significant. Statistical analyses were performed with GraphPad Prism software.

Results are shown on Fig. 10.

The adhesion of keratinocytes to the three cross-linked gels is at least as good as on gelatin alone, known to be a good adhesion substrate for keratinocytes. Cell adhesion to the reticulated gels is progressive and correlated to the level of cross-link.

Thus, crosslinking of gelatin does not alter keratinocyte interaction with gelatin fibers. Therefore, the network according to the invention can be used as a support for keratinocyte (re)covering of a wound, with an efficiency similar to a natural epidermal regeneration process.

**Example** 4 - **Synthesis of compounds according to the invention**

The inventor's objective was to produce bio-sourced and valid alternative to CYN. CYN-like derivatives based on bio-sourced and commercially available platform molecules, such as citral and citronellal, to yield new crosslinking agents in sufficient amounts.

As previously reported on n-pentanal and 2-cyclohexylacetaldehyde model compounds a simple Horner-Wadsworth-Emmons (H.W.E.) reaction of citral (1) or citronellal (2) with phosphonoester (3) will afford an E/Z mixture of olefinic diesters. Subsequent separation of the two isomers, reduction of each with LiAIH4, and oxidation with Dess-Martin Periodinane (DMP) will yield CYN-like dialdehydes (1a), (1b), (2a), and (2b) as described in Scheme 1.

Pure compounds have been subjected to different spectroscopic techniques including NMR spectroscopy (1D, 1H, 13C, DEPT 135; 2D, 1H-1H COSY, 1H-13C HSQC, 1H-13C HMBC, 1H-1H ROESY/1H-1H NOESY) and HRMS spectrometry.

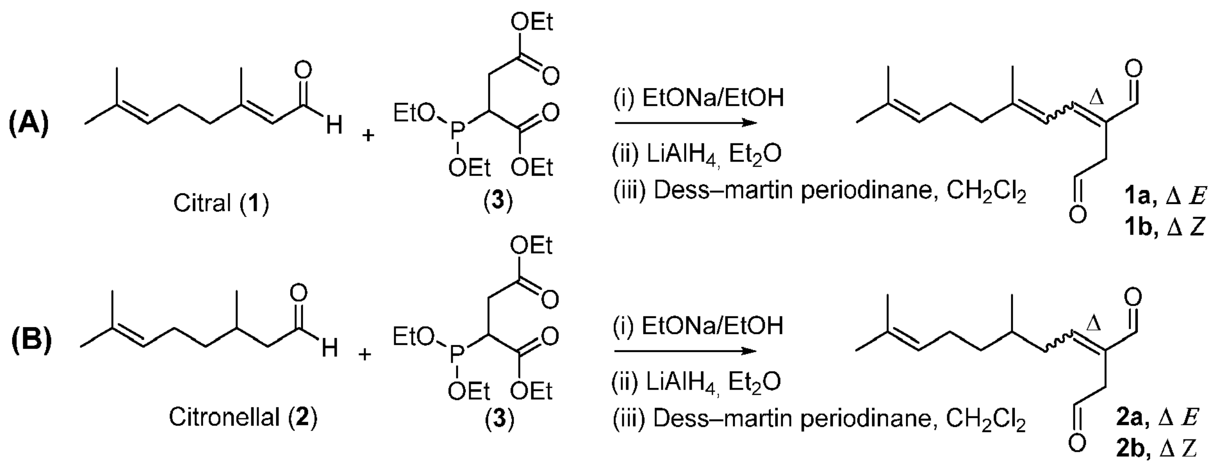


Scheme 1. Synthetic route to CNY-like derivatives.

More generally, the compounds according to the invention of formula II, are obtained according to the following reaction

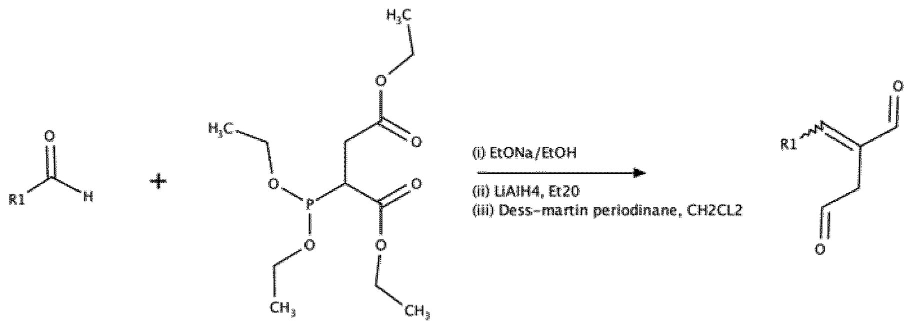


## Claims

**1.** Composition comprising a homogenous mixture:

- a polymer containing at least two free amino functional groups, and
- a compound comprising an aldehyde functional group, said compound being of formula I

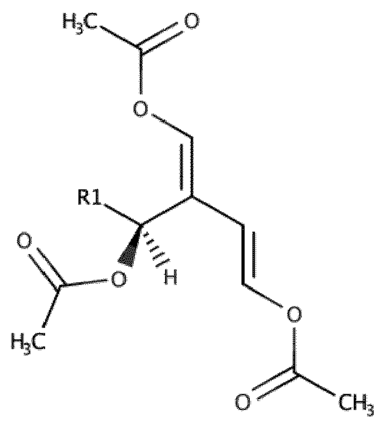
(I);

or of formula II,

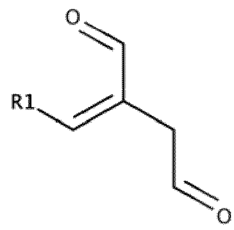

(II),

wherein R1 is C5-C25 alkyl, having one or more double bonds, and/or one or more triple bonds, possibly branched,
wherein the stoichiometric ratio between the aldehyde functional group contained in the compound and the amino functional groups contained in the polymer is from 0.1 to 3.

2. A structured network comprising or consisting essentially of a polymer containing at least two free amino functional groups operably crosslinked to compound comprising two aldehyde groups of formula II,

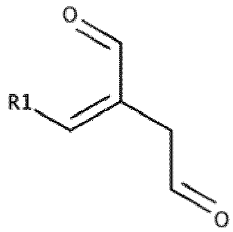

(II),

wherein R1 is C5-C25 alkyl, having one or more double bonds, and/or one or more triple bonds, possibly branched, and
wherein the stoichiometric ratio between the aldehyde functional group contained in the compound and the amino functional groups contained in the polymer is from 0.1 to 3,
or an hydrated form thereof.

3. The structured network according to 2, wherein the polymer containing at least two free amino functional groups is an animal or plant protein, a polysaccharide containing an amino group or an artificial amino polymer.

4. The structured network according to claim 3, wherein the animal or plant protein is chosen from gelatin, keratin, casein and lactoserum proteins, elastin, collagen, soy protein, serum bovine albumin, fibrin and fibrinogen, and resilin.

5. The structured network according to claim 2 or 3, wherein the compound of formula II is oxytoxin-2 of formula III

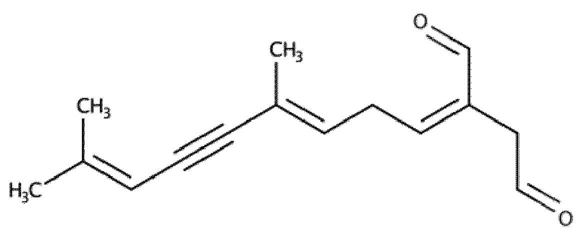

(III).

6. The structured network according to any one of claims 2 to 5, wherein the polymer containing at least two free amino functional groups is chosen from gelatin and chitosan.

7. Use of a composition according to claim 1 or a structured network according to anyone of claims 2 to 6, for lubricating a hydrophobic surface of a device, in particular a medical device.

**8.** A structured network according to any one of claims claim 2 to 5, for its use for treating an animal wound.

**9.** A medical device comprising a surface, the medical device comprising a structured network according to anyone of claims 2 to 5 coated to the surface.

**10.** The medical device according to claim 9, said medical device being a wound dressing; a patch; a film; a wound filler; an hydrogel dressing; a skin substitute; an hydrocoilloid dressing or a foam; a carrier for cells, molecules, genes, bioactive compounds or nanoparticles; and a scaffold for organoid culture.

**11.** A method for producing a structured network, the method comprising:

- mixing

  - a first organic solvent comprising a polymer containing at least two free amino functional groups; and
  - a second organic solvent comprising a compound containing comprising 2 aldehyde groups of formula I,

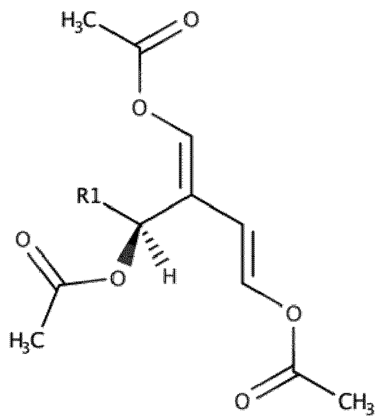

(I),

to obtain a mixture

wherein the stoichiometric ratio between the aldehyde functional group contained in the compound and the amino functional groups contained in the polymer is from 0.1 to 3,
wherein said first organic solvent is compatible with said second solvent,

- contacting the mixture with a basic composition, to obtain a crosslinked mixture,
- possibly washing the crosslinked mixture, and
- recovering the crosslinked mixture which is in a form of a structured network.

**12.** The method for producing a structured network according to claim 11, wherein the mixture is poured in a recipient and cooled down to a temperature from 2°C to 15°C, and wherein the polymer containing at least two free amino functional groups is gelatin.

**13.** A structured network obtained or liable to be obtained by the method according to any one of claims 11 to 12.

**14.** A kit comprising

- a polymer containing at least two free amino functional groups as defined in claim 1, 3, 4, or 6; and
- a compound of formula I

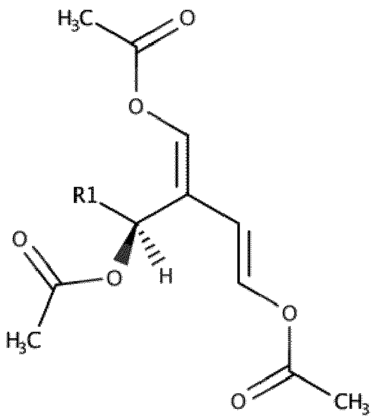

,

Wherein R1 is a C5-C25 terpen;
- and possibly an aqueous basic solution.

**15.** Use of a compound

- of formula I as defined in claim 1, or
- of formula II as defined in claim 1 or 5,

for crosslinking, preferably *in vitro* or *ex vivo*, a polymer containing at least two free amino functional groups, in order to obtain a structured network.

[Fig. 1]

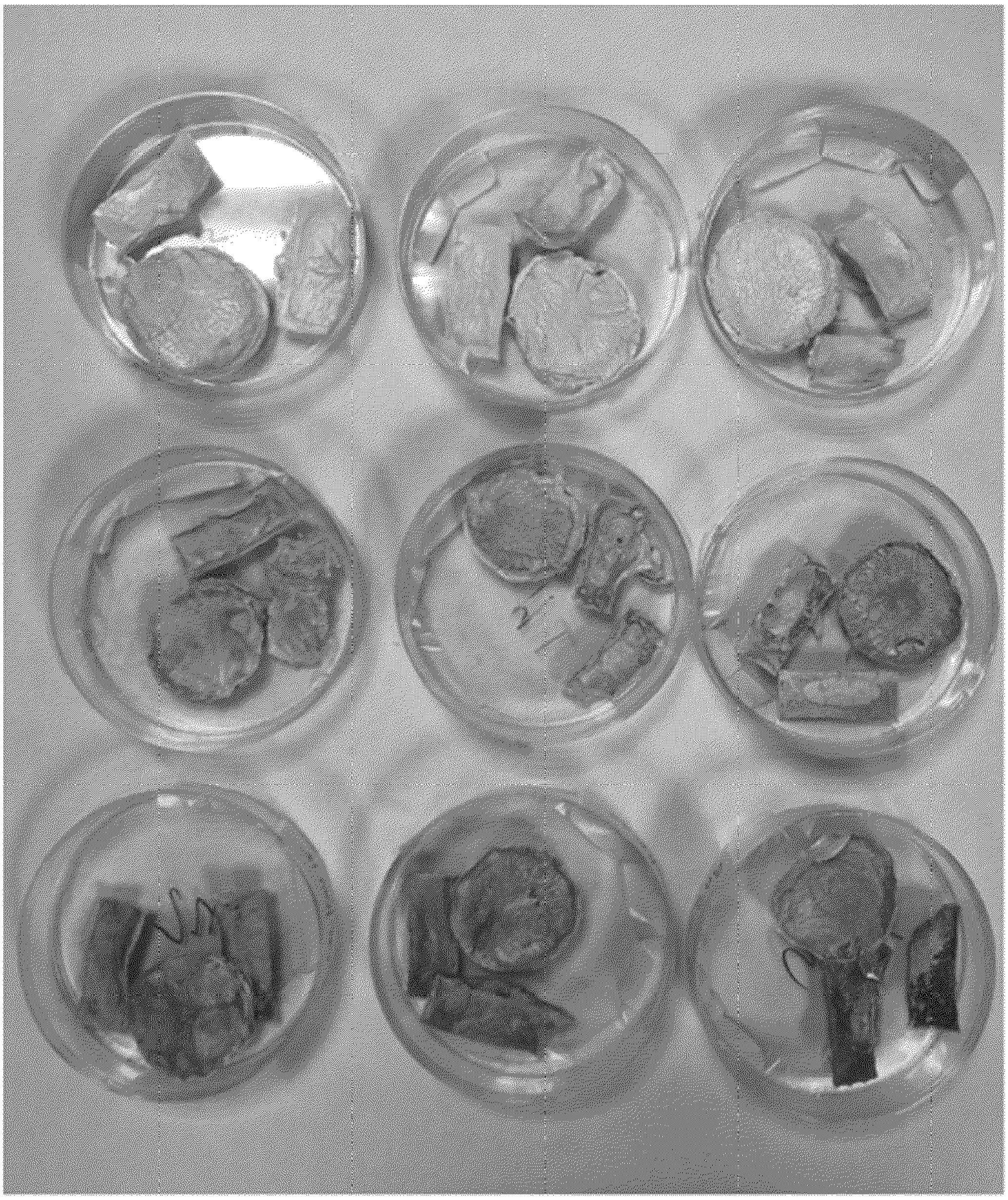

A

B

C

[Fig. 2]

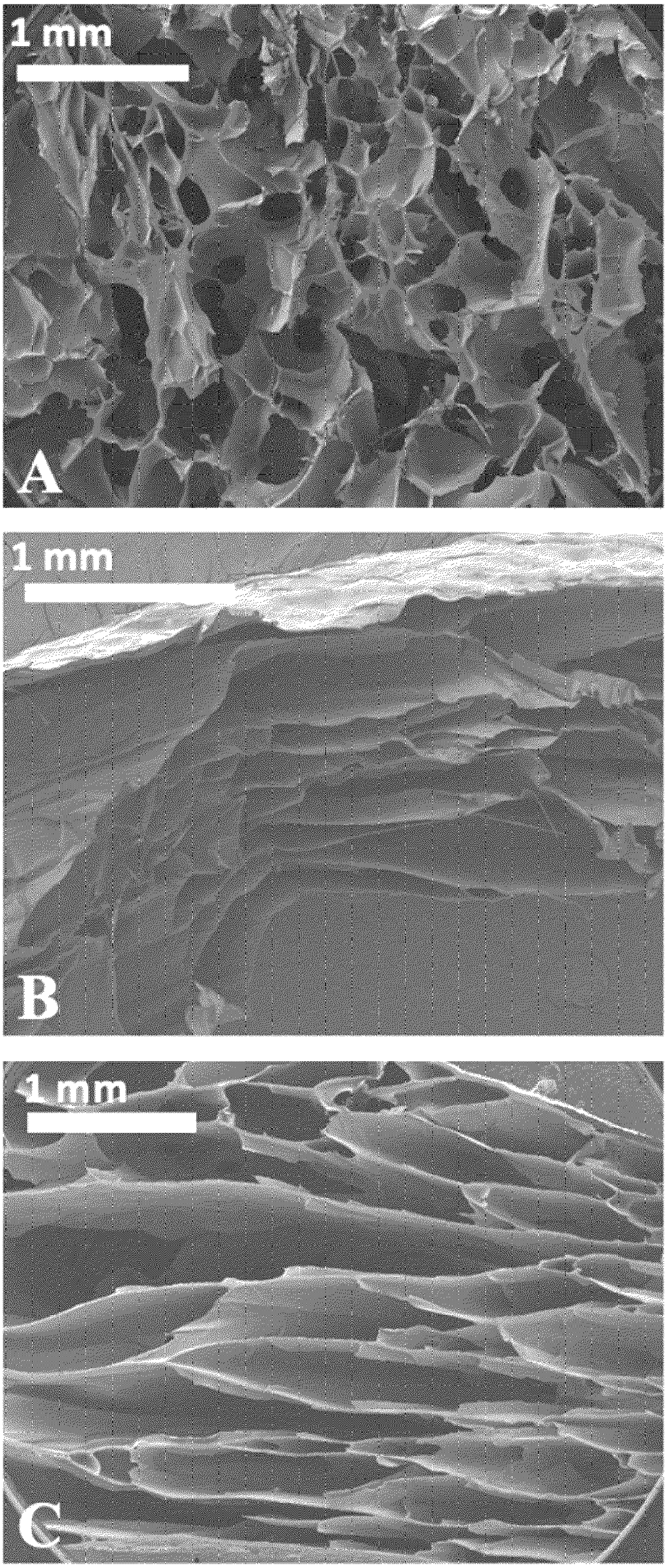

[Fig. 3]

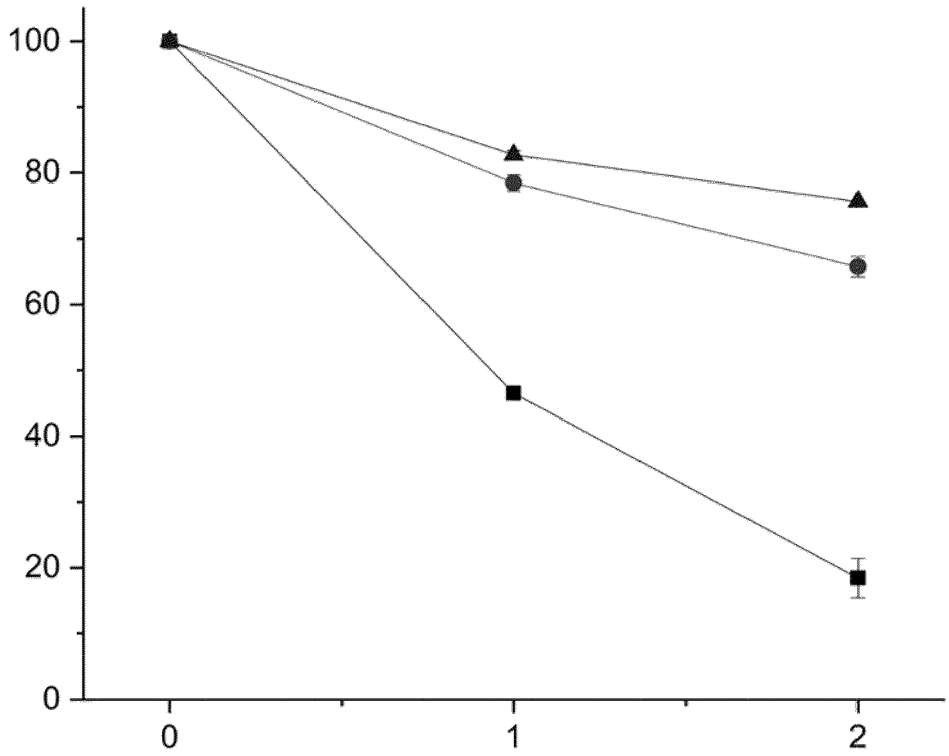


[Fig. 4]

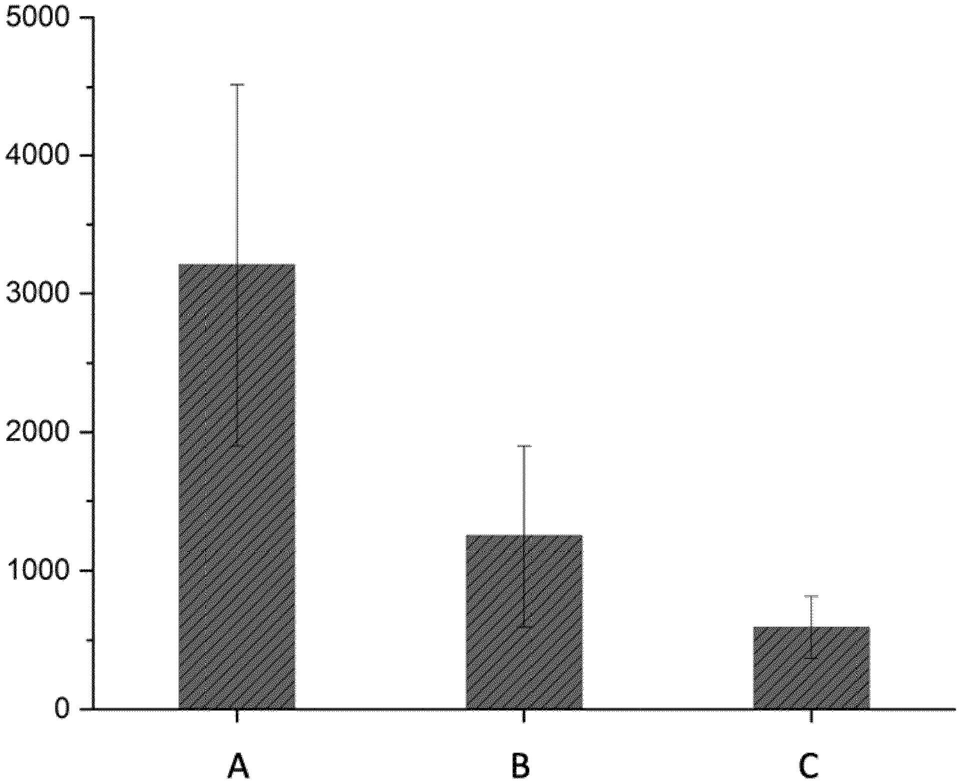

[Fig. 5]

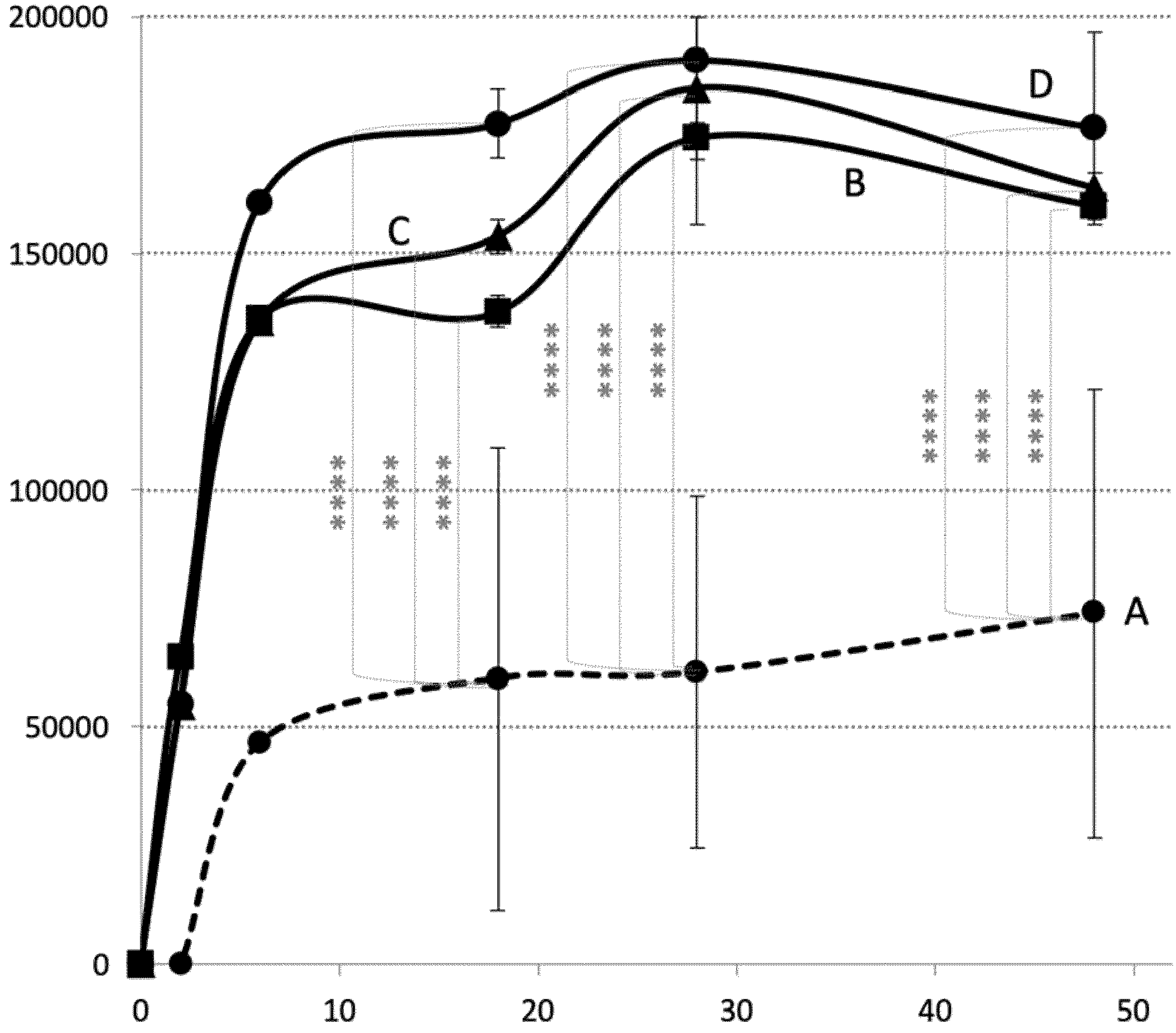


[Fig. 6]

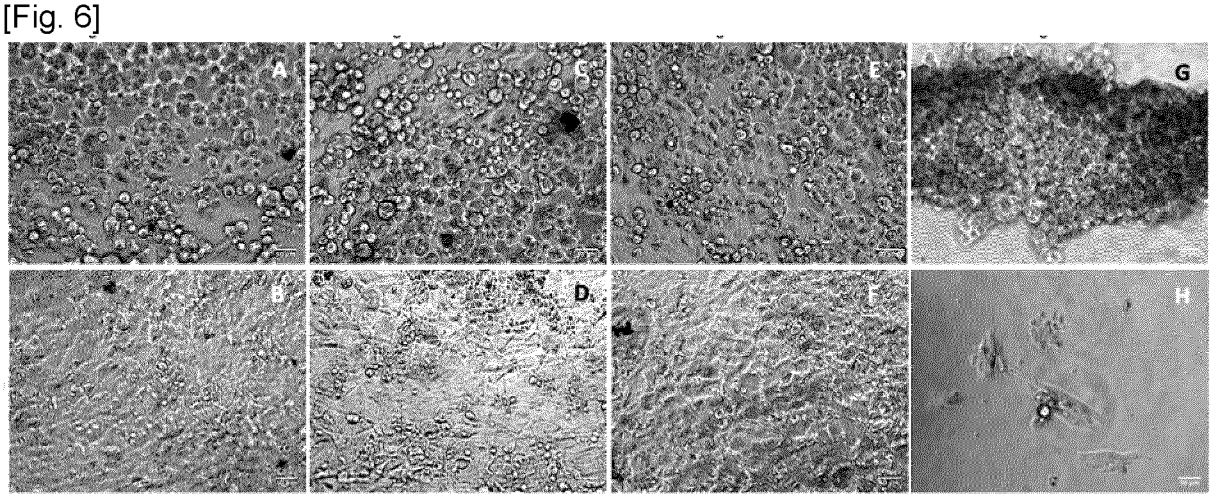

[Fig. 7]

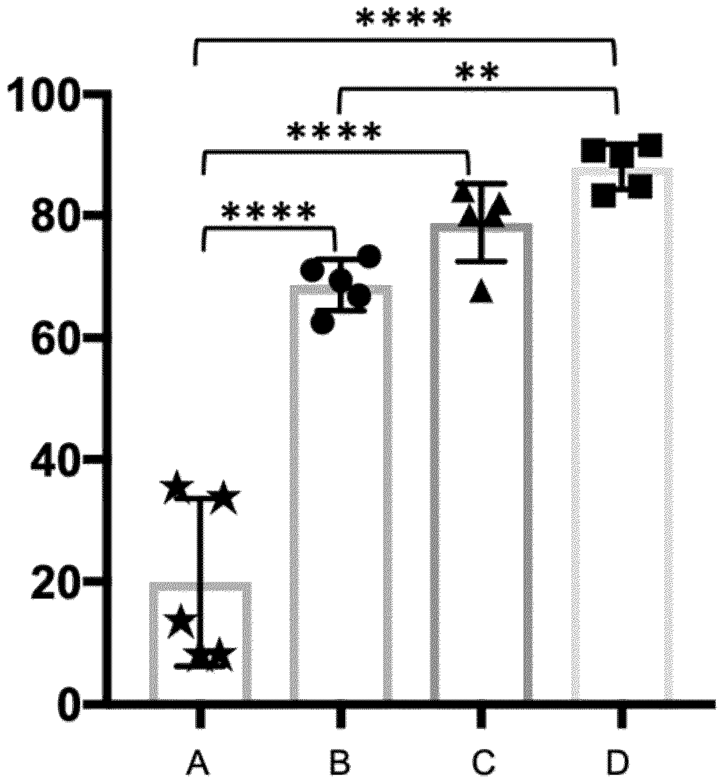
****
**
****
****
100
80
60
40
20
0
A
B
C
D

[Fig. 8]

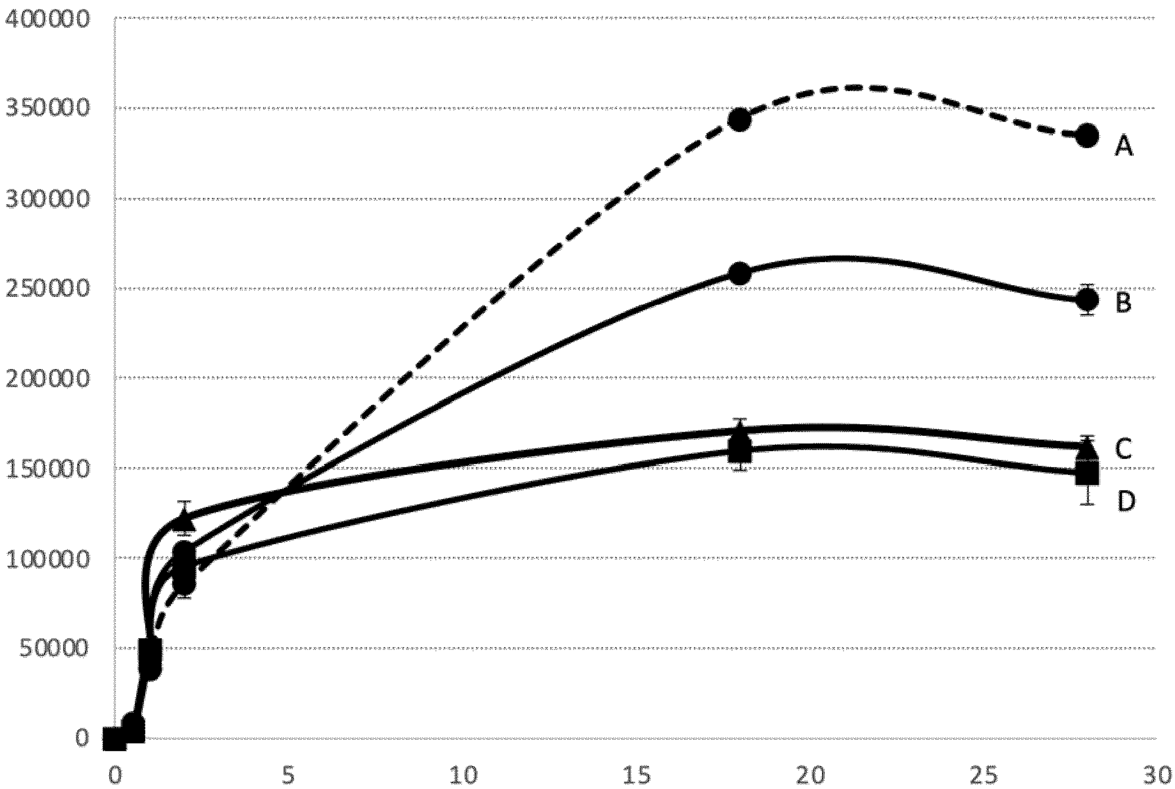
400000
350000
300000
250000
200000
150000
100000
50000
0
0
5
10
15
20
25
30
A
B
C
D

[Fig. 9]

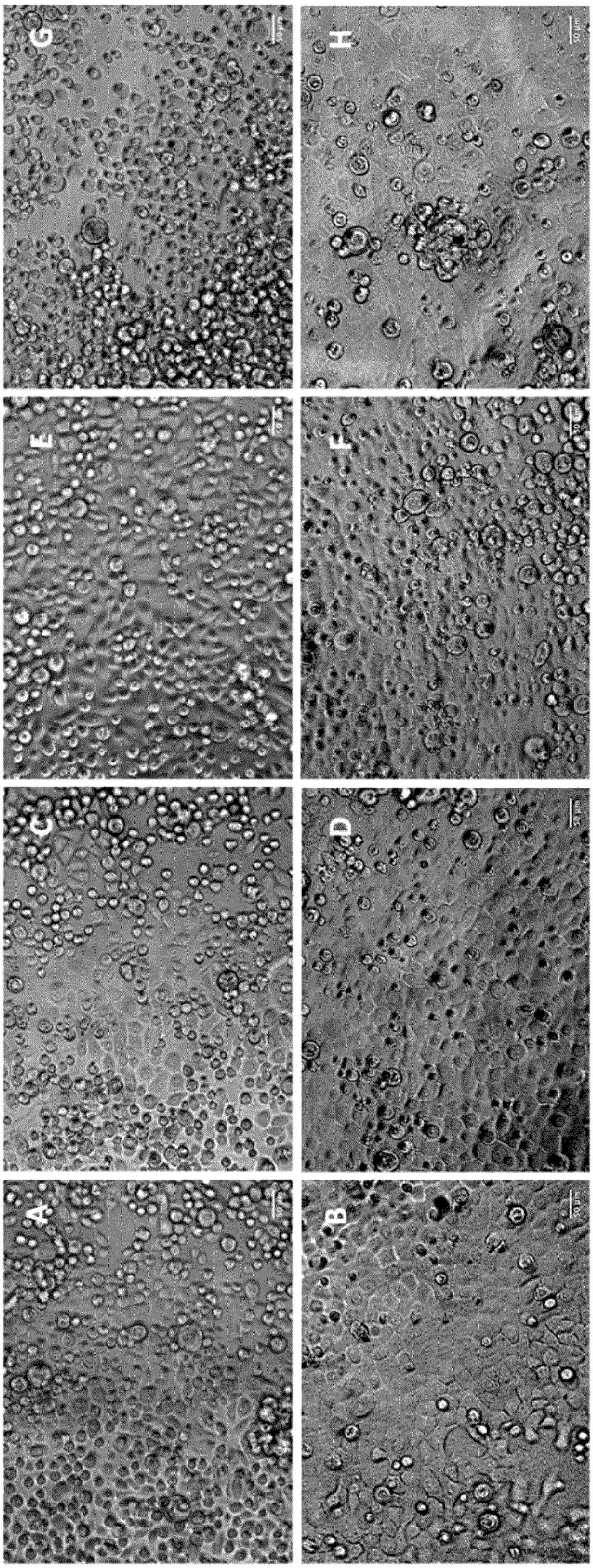

[Fig. 10]

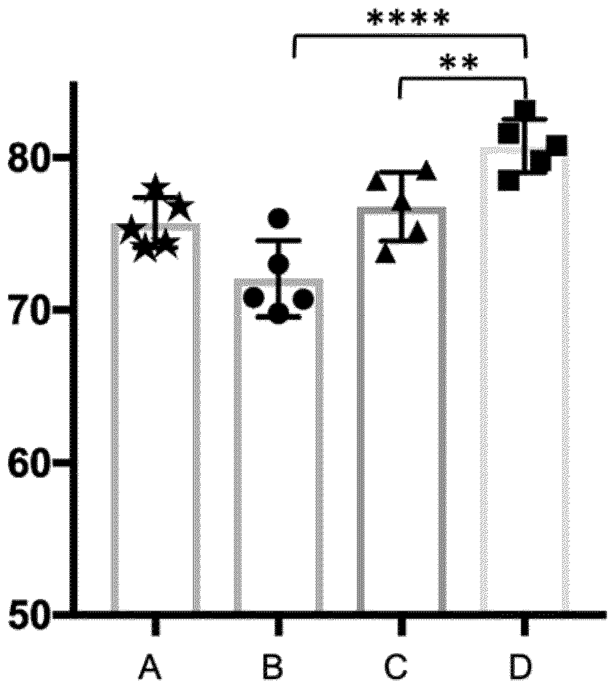

Europäisches Patentamt
European Patent Office
Office européen des brevets

# EUROPEAN SEARCH REPORT

Application Number
EP 22 30 6657

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br><br><br><br><br><br>Y | CADELIS MELISSA M ET AL: "Investigation of the electrophilic reactivity of the biologically active marine sesquiterpenoid onchidal and model compounds",<br>BEILSTEIN JOURNAL OF ORGANIC CHEMISTRY, vol. 14, 2 August 2018 (2018-08-02), pages 2229-2235, XP093035110,<br>DOI: 10.3762/bjoc.14.197<br>Retrieved from the Internet:<br>URL:https://www.beilstein-journals.org/bjoc/content/pdf/1860-5397-14-197.pdf><br>* page 2230; figure 1 *<br>* page 2231; figure 2 *<br>* page 2233; table 1 *<br>----- | 1-3,13,<br>15<br><br><br><br><br><br><br><br><br><br>1-15 | INV.<br>A61L15/32<br>C08H1/02<br>C08H1/06<br>C08J3/24 |
| X | Cengiz Sevilay ET AL: "A Low Cost Immobilization Agent From an Invasive Marine Alga: Caulerpa racemosa var. cylindracea Biomass In Bovine Serum Albumin Immobilization",<br>Turkish Journal of Biochemistry, 2008, 33(2),<br>23 July 2008 (2008-07-23), pages 64-70, XP093035111,<br>Retrieved from the Internet:<br>URL:https://www.researchgate.net/publication/265614012_A_Low_Cost_Immobilization_Agent_From_an_Invasive_Marine_Alga_Caulerpa_racemosa_var_cylindracea_Biomass_In_Bovine_Serum_Albumin_Immobilization<br>* pages 388, 389 *<br>----- | 1-5,11,<br>13,15 | TECHNICAL FIELDS SEARCHED (IPC)<br>C08H<br>A61L<br>C08J |
| Y | EP 2 771 027 B1 (BAXTER INT [US]; BAXTER HEALTHCARE SA [CH])<br>19 August 2015 (2015-08-19)<br>* paragraphs [0006], [0016] *<br>-----<br>-/-- | 1-15 | |

The present search report has been drawn up for all claims

1

| Place of search | Date of completion of the search | Examiner |
|---|---|---|
| Munich | 31 March 2023 | Arz, Marius |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 3

Europäisches Patentamt
European Patent Office
Office européen des brevets

# EUROPEAN SEARCH REPORT

Application Number
EP 22 30 6657

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | IMOTO T. ET AL: "Electrostatic free energy of lysozyme", BIOPHYSICAL JOURNAL, vol. 44, no. 3, 1 December 1983 (1983-12-01), pages 293-298, XP093035523, AMSTERDAM, NL ISSN: 0006-3495, DOI: 10.1016/S0006-3495(83)84302-1 * page 293 * ----- | 1 | |
| A | CAVAS LEVENT ET AL: "The Potential of Caulerpa spp. for Biotechnological and Pharmacological Applications", 1 January 2010 (2010-01-01), SEAWEEDS AND THEIR ROLE IN GLOBALLY CHANGING ENVIRONMENTS, SPRINGER, DORDRECHT, NL, PAGE(S) 385, 387 - 397, XP009143632, ISBN: 978-90-481-8569-6 * page 388 - page 389 * ----- | 1 | TECHNICAL FIELDS SEARCHED (IPC) |
| L | Anonymous: "BSA; Bovine Serum Albumin", , 1 March 2023 (2023-03-01), pages 1-1, XP093035530, Retrieved from the Internet: URL:https://ca.vwr.com/assetsvc/asset/en_CA/id/8040115/contents * page 1 * ----- -/-- | 1 | |

The present search report has been drawn up for all claims

1

| Place of search | Date of completion of the search | Examiner |
|---|---|---|
| Munich | 31 March 2023 | Arz, Marius |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 22 30 6657

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PETROS SEBLEWONGEL ET AL: "A Review on Gelatin Based Hydrogels for Medical Textile Applications", JOURNAL OF ENGINEERING, vol. 2020, 22 December 2020 (2020-12-22), pages 1-12, XP093035519, ISSN: 2314-4904, DOI: 10.1155/2020/8866582 Retrieved from the Internet: URL:http://downloads.hindawi.com/journals/je/2020/8866582.xml> * page 8 - page 10 * ----- | 6-10,12, 14 | |

TECHNICAL FIELDS SEARCHED (IPC)

The present search report has been drawn up for all claims

1

| Place of search | Date of completion of the search | Examiner |
|---|---|---|
| Munich | 31 March 2023 | Arz, Marius |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

# ANNEX TO THE EUROPEAN SEARCH REPORT ON EUROPEAN PATENT APPLICATION NO.

EP 22 30 6657

This annex lists the patent family members relating to the patent documents cited in the above-mentioned European search report. The members are as contained in the European Patent Office EDP file on The European Patent Office is in no way liable for these particulars which are merely given for the purpose of information.

31-03-2023

| Patent document cited in search report | Publication date | Patent family member(s) | Publication date |
|---|---|---|---|
| EP 2771027 B1 | 19-08-2015 | AR 088531 A1 | 18-06-2014 |
| | | AU 2012330450 A1 | 29-05-2014 |
| | | BR 112014009986 A2 | 30-05-2017 |
| | | CA 2853356 A1 | 02-05-2013 |
| | | CL 2014001063 A1 | 27-02-2015 |
| | | CN 103889447 A | 25-06-2014 |
| | | CO 6970594 A2 | 13-06-2014 |
| | | DK 2771027 T3 | 02-11-2015 |
| | | EP 2771027 A1 | 03-09-2014 |
| | | ES 2553702 T3 | 11-12-2015 |
| | | HK 1194968 A1 | 31-10-2014 |
| | | JP 6038164 B2 | 07-12-2016 |
| | | JP 2014530889 A | 20-11-2014 |
| | | KR 20140082841 A | 02-07-2014 |
| | | MX 347623 B | 04-05-2017 |
| | | NZ 623908 A | 28-08-2015 |
| | | PL 2771027 T3 | 29-01-2016 |
| | | PT 2771027 E | 26-11-2015 |
| | | RU 2014121232 A | 10-12-2015 |
| | | SG 11201401878S A | 26-09-2014 |
| | | TW 201332566 A | 16-08-2013 |
| | | US 2013108671 A1 | 02-05-2013 |
| | | US 2016354511 A1 | 08-12-2016 |
| | | UY 34414 A | 30-04-2013 |
| | | WO 2013060770 A1 | 02-05-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6132759 A

### Non-patent literature cited in the description

- **PARENT-MASSIN.** *Journal of Toxicology and Environmental Health,* 1996, vol. 47 (1), 47-59